# EUROPÄISCHE PATENTSCHRIFT

(11) **EP 2 179 269 B1**
(45) Veröffentlichungstag und Bekanntmachung des Hinweises auf die Patenterteilung: **27.01.2016**
(21) Anmeldenummer: 08801554.0
(22) Anmeldetag: 09.08.2008
(51) Int. Cl.: G01N 21/45, G01N 21/84, G01N 33/543, G01N 33/551

(54) **UNTERSUCHUNG MOLEKULARER WECHSELWIRKUNGEN AN UND/ODER IN DÜNNEN SCHICHTEN**
ANALYSIS OF MOLECULAR INTERACTIONS ON AND/OR IN THIN LAYERS
RECHERCHE D'INTERACTIONS MOLECULAIRES SUR ET/OU DANS DES COUCHES MINCES

(30) Priorität: 09.08.2007 DE 102007038797
(43) Veröffentlichungstag der Anmeldung: 28.04.2010
(73) Patentinhaber: Biametrics GmbH, 72076 Tübingen (DE)
(72) Erfinder: GAUGLITZ, Günter, 72070 Tübingen (DE); PROLL, Günther, 73770 Denkendorf (DE); PRÖLL, Florian, 68165 Mannheim (DE); STEINLE, Lutz, 88094 Oberteuringen (DE); SCHUBERT, Markus, 72074 Tübingen (DE)
(74) Vertreter: Wüstefeld, Regine Marie
(86) Internationale Anmeldenummer: PCT/EP2008/006587
(87) Internationale Veröffentlichungsnummer: WO 2009/019043

(56) Entgegenhaltungen:
- DE-A1- 19 830 727
- DE-A1-102005 015 030
- GB-A- 2 416 621
- US-A1- 2003 222 223
- US-A1- 2006 152 833
- US-A1- 2006 197 960
- JÜRGEN KÖHLER, UWE RAU, MARCUS B. SCHUBERT, JÜRGEN H. WERNER: "Photons and photonics in solar cells and photodiodes" THEMENHEFT FORSCHUNG, Nr. 2, 2005, Seiten 96-103, XP002513673 Stuttgart & [Online] ISSN: 1861-0269 Gefunden im Internet: URL:http://www.uni-stuttgart.de/zv/themenh eft/02/index.html> [gefunden am 2009-02-03]
- HSIUNG-KUANG TSAI, SI-CHEN LEE, WEI-LIANG LIN: "An amorphous SiC/Si two-color detector" IEEE ELECTRONIC DEVICE LETTERS, Bd. EDL-8, Nr. 8, 15. August 1987 (1987-08-15), Seiten 365-367, XP002513674

## Beschreibung

Die Erfindung betrifft einen Träger für eine dünne Schicht und ein Verfahren zur Untersuchung molekularer Wechselwirkungen an und/oder in einer solchen dünnen Schicht.

Wechselwirkungen biologisch aktiver Moleküle nehmen eine zentrale Stellung in biochemischen Prozessen ein. Beispielhaft seien Genexpression, Signalübertragung durch Hormone oder Neurotransmitter, Immunabwehr durch Antigen-Antikörper-Bindung oder Enzymreaktionen genannt. Jedem dieser Vorgänge geht die spezifische Bindung eines Liganden an einen biologischen Rezeptor voraus. Der Nachweis und die Charakterisierung solcher Bindungsereignisse sind für die Aufklärung und das Verständnis von Mechanismus und Funktion biochemischer Prozesse von entscheidender Bedeutung.

Herkömmliche Verfahren zur Charakterisierung biomolekularer Erkennungsreaktionen basieren meist auf einer radioaktiven Markierung oder einer Fluoreszenz-Markierung eines der beiden Bindungspartner. Nachteil dieser Methoden ist, daß mit der Markierung eine aufwendige und zeitraubende Aufreinigung des markierten Bindungspartners verbunden ist und der Erhalt von dessen voller biologischer Funktion nicht garantiert werden kann. Daher wurden im Verlauf der letzten Jahre verschiedene Verfahren zur markierungsfreien Detektion biomolekularer Wechselwirkungen an Festphasen untersucht und geeignete Detektionstechniken entwickelt, die die direkte Beobachtung von Bindungsereignissen an Oberflächen und damit den Zugang zur thermodynamischen und kinetischen Charakterisierung von Affinitätsreaktionen erlauben.

Zur markierungsfreien Detektion biomolekularer Wechselwirkungen sind insbesondere Verfahren basierend auf der Reflektometrischen Interferenzspektroskopie (RIfS) geeignet. Diese optische Detektionsmethode macht sich die Mehrfachreflexion von elektromagnetischer Strahlung an dünnen, transparenten Schichten zunutze. Einfallende elektromagnetische Strahlung wird teilweise an den Begrenzungsflächen (Phasengrenzen) der Schichten aufgrund unterschiedlicher Brechungsindices reflektiert. Durch die Überlagerung der reflektierten Strahlung wird ein charakteristisches Interferenzmuster erzeugt (A. Brecht, G. Gauglitz, W. Nahm, Analysis 20 (1992) 135; A. Brecht, G. Gauglitz, G. Kraus, W. Nahm, Sens. Actuators 11B (1993) 21; Brecht et al., Optical probes and transducers, Biosensors & Bioelectron. 10 (1995), 923-936; Schmitt et al., An integrated system for optical biomolecular interaction analysis, Biosensors & Bioelectron. 12 (1997), 809-816). Die Änderung der optischen Schichtdicke der untersuchten dünnen Schicht, die z.B. mit der Anlagerung oder der Bindung von Molekülen verbunden sein kann, verursacht eine Verschiebung des Interferogramms. Diese Verschiebung kann zeitaufgelöst gemessen werden. RIfS eignet sich somit hervorragend z.B. zur Untersuchung von Bindungsereignissen an einer Oberfläche.

Ein zur Untersuchung physikalischer, chemischer und/oder biochemischer Reaktionen und Wechselwirkungen geeignetes Verfahren basierend auf diesem Prinzip ist z. B. aus DE-A-198 30 727 bekannt. Allerdings erfordert die dort beschriebene Vorgehensweise spezielle, optisch vergütete Träger. Zudem ist das beschriebene Verfahren relativ aufwendig in der Handhabung, und es liefert häufig ein relativ schlechtes Signal/Rausch-Verhältnis.

In der DE-A-10 2005 015 030 wird ein RIfS-Verfahren beschrieben, bei dem die zu messende dünne Schicht mittels einer Lichtquelle mit Licht bestrahlt und der an den Begrenzungsflächen der dünnen Schicht reflektierte Strahlungsanteil an einem Detektor gemessen wird, wobei gezielte Maßnahmen getroffen werden, daß am Detektor nur monochromatisches Licht einer einzigen Wellenlänge (oder eines sehr schmalen Spektrums) ankommt. Dies wird insbesondere dadurch erreicht, daß die verwendete Lichtquelle monochromatisches Licht ausstrahlt. Durch den Einsatz von extrem schmalbandigen Lasern und die Selektion einer geeigneten Wellenlänge läßt sich eine deutliche Verbesserung des Signal/Rausch-Verhältnisses erzielen. Allerdings muß dabei ein sehr ungünstiges Kosten/Nutzen-Verhältnis in Kauf genommen werden, da die zur Durchführung des Verfahrens benötigten Geräte sehr teuer sind. Zudem müssen Schichten mit vorgegebener Schichtdicke bereitgestellt werden, was sich in der Praxis als sehr nachteilig erwiesen hat.

In einem wissenschaftlichen Artikel der Herren Jürgen Köhler, Uwe Rau, Marcus B. Schubert und Jürgen H. Werner, der in dem Themenheft Forschung, Nr. 2, 2005 auf den Seiten 96 - 103 unter dem Titel "Photons and photonics in solar cells and photodiodes" veröffentlicht worden und unter der Chiffre XP002513673 Stuttgart, abrufbar ist, wird ein Ausblick auf zukünftige Anwendungen der sogenannten "Thin-Film-on-CMOS-Technologie" gegeben, in dem Artikel kurz TFC-Technologie genannt. Darunter ist eine spezielle Bildsensor-Technologie zu verstehen, bei welcher Photodetektoren auf der Basis amorphen Siliziums verwendet werden, welche bei niedrigen Temperaturen auf die mikroelektronischen Ausleseschaltungen eines Bildsensors aufgebracht werden. In dem Artikel wird als Ausblick auf zukünftige Anwendungen die Möglichkeit angesprochen, mit dieser TFC-Technologie eine erleichterte Abstimmung der spektralen Resonanz bei Verwendung einer Dünnschicht-Photodiode auf der Basis von Si:H zu nutzen. Die Autoren gehen davon aus, daß sich mögliche Anwendungen z.B. bei der Untersuchung von Bioanalyten ergeben können, wenn eine einfache Photodiode vom p-in - Typ durch weiterentwickelte Photodioden mit einer n -i-p-i-n - oder p-i--i n - Struktur ersetzt wird. Auf diese Weise soll es möglich sein, die spektrale Empfindlichkeit durch eine Veränderung der Auslesespannung dynamisch anpassen zu können. Konkrete Anregungen zu einer Umsetzung dieser gedanklichen Möglichkeiten gibt der Artikel nicht.

Als weiterer Stand der Technik ist noch die US 2006/197960 A1 zu erwähnen, welche einen Biochip für die DNA- bzw. Proteinanalyse offenbart, der eine vereinfachte Analyse erlauben soll, weil er markierungsfrei arbeitet, d.h. frei von bisher üblichen Fluoreszenzmarkern, und mit einer Vielzahl von möglichen Bindungsstellen für die Biomoleküle. Mit diesem Biochip soll z.B. die direkte Beobachtung des Hybridisierungsstatus von DNA durch Vergleich mit einem Referenz-Lichtwellenleiter ermöglicht werden. Für diesen Biochip wird ein Träger verwendet, der eine Schutzschicht aus Siliziumdioxid aufweisen kann und auf dem zumindest eine Sensorschicht mit optoelektronischen Eigenschaften angeordnet ist. Die Sensorschicht umfaßt eine Halbleiterschicht mit der Schichtfolge p-n.

Der vorliegenden Erfindung liegt die Aufgabe zugrunde, ein Verfahren zur Untersuchung molekularer Wechselwirkungen bereitzustellen, das nicht von vorgegebenen Probenbedingungen abhängig ist und insbesondere auch kostengünstig ist. Darüber hinaus soll das neue Verfahren den aus dem Stand der Technik bekannten Lösungen besonders im Hinblick auf das erzielbare Signal/Rausch-Verhältnis überlegen sein. Der vorliegenden Erfindung liegt des weiteren die Aufgabe zugrunde, einen für ein solches Verfahren geeigneten Träger bereitzustellen.

Diese Aufgabe wird zunächst gelöst durch ein Verfahren zur Untersuchung molekularer Wechselwirkungen an und/oder in einer auf einem Träger angeordneten dünnen Schicht mittels der Reflektometrischen Interferenzspektroskopie (RIfS), wobei das Verfahren die nachfolgend genannten Schritte umfaßt. In einem Schritt wird die dünne Schicht mit elektromagnetischer Strahlung von mindestens einer Strahlungsquelle beleuchtet. Die dünne Schicht weist Begrenzungsflächen auf, an denen die elektromagnetische Strahlung reflektiert werden kann. Die Begrenzungsflächen sind vorzugsweise im wesentlichen eben und sind insbesondere parallel zueinander angeordnet. In einem weiteren Schritt der erfindungsgemäßen Verfahrens wird mittels mindestens eines optoelektronischen Wandlers auf der Basis eines amorphen, nanokristallinen oder polykristallinen Si-basierten Halbleitermaterials ein an Begrenzungsflächen der dünnen Schicht reflektierter Strahlungsanteil erfaßt, und die erfaßte Strahlung in einen frequenz- und intensitätsabhängigen Photostrom umgewandelt. Zur Messung des Photostroms wird an den optoelektrischen Wandler eine Auslesespannung angelegt.
Die spektrale Empfindlichkeit des optoelektrischen Wandlers wird durch Veränderung der Auslesespannung derart variiert, daß ein im wesentlichen konstanter Photostrom erhalten wird. Dieser Vorgang wird im folgenden auch als Nullabgleich bezeichnet. Dabei werden aus der Auslesespannung Parameter abgeleitet, welche die zu untersuchenden Wechselwirkungen an und/oder in der dünnen Schicht kennzeichnen.

Im Unterschied zu aus dem Stand der Technik bekannten Verfahren werden also gemäß der vorliegenden Erfindung Parameter bevorzugt nicht etwa unmittelbar aus einer Änderung eines Photostroms gewonnen. Stattdessen ist es bevorzugt, durch Variation der angelegten Auslesespannung den Photostrom konstant zu halten. Veränderungen der Schichtdicke führen in der Regel zu einer Verschiebung der Flanke des Interferenzspektrums über die Detektionskurve hinweg und zu einer Änderung der Intensität des Detektorsignals. Erfindungsgemäß kann durch Änderung der Auslesespannung der Wendepunkt der sinusförmigen Detektionskurve in den Wendepunkt des Interferenzspektrums verschoben werden. Im Gegensatz zu den bekannten Verfahren muß so bei Beginn der Untersuchung die Ausgangsschichtdicke nicht mehr festgelegt werden. Darüber hinaus erlaubt die erfindungsgemäße Vorgehensweise präzise Reaktionen bereits auf minimale Änderungen der Strahlungsintensität und damit des Photostroms. Strommessung und Spannungsvorgabe können so genau gestaltet werden, daß auf diese Weise das Signal/Rausch-Verhältnis wesentlich verbessert wird. Ein Nullabgleich läßt sich vergleichsweise sehr viel exakter durchführen als die Extraktion eines absoluten Photostromsignals aus Hintergrundrauschen.

Die mit elektromagnetischer Strahlung beleuchtete dünne Schicht ist in besonders bevorzugten Ausführungsformen des erfindungsgemäßen Verfahrens auf einem vorzugsweise festen Träger angeordnet. Bei dem Träger kann es sich beispielsweise um ein flächiges Substrat aus Glas, Kunststoff oder Metall handeln.

Vorzugsweise ist die auf dem Träger angeordnete dünne Schicht der einfallenden Strahlung abgewandt. Dann muß das Trägermaterial zumindest teilweise für die verwendete elektromagnetische Strahlung durchlässig sein. Weiter bevorzugt erfolgt die Beleuchtung der dünnen Schicht durch den Träger hindurch.

Unter dem Begriff "dünne Schicht" wird vorliegend insbesondere eine Schicht verstanden, deren Dicke zwischen 0,1 nm und 100 µm liegt. Innerhalb dieses Bereiches kann die Schichtdicke und gegebenenfalls auch die Schichtdichte während des erfindungsgemäßen Verfahrens variieren.

Bei der dünnen Schicht handelt es sich vorzugsweise um eine gegebenenfalls mehrlagige Schicht aus organischen Molekülen. Vorzugsweise umfaßt die dünne Schicht mindestens eine Lage aus Molekülen, die auf dem Träger immobilisiert sind. Mit anderen Worten, der Träger weist also eine Oberfläche auf, die mit darauf immobilisierten Molekülen modifiziert ist. Die Eigenschaften der Oberfläche werden durch die Eigenschaften der immobilisierten Moleküle bestimmt.

Bei den zu untersuchenden molekularen Wechselwirkungen handelt es sich vorzugsweise um physikalische, chemische und/oder biochemische Reaktionen, Erkennungs-, Bindungs- und/oder Anlagerungsereignisse. Insbesondere handelt es sich bei den zu untersuchenden molekularen Wechselwirkungen um Wechselwirkungen zwischen den immobilisierten Molekülen auf der Oberfläche des Trägers und Molekülen aus einer Probe (kurz: Probenmoleküle), die vor und/oder während dem Beleuchten mit der dünnen Schicht in Kontakt gebracht wird. Es ist bevorzugt, daß die Probe dazu mit den immobilisierten Molekülen des Trägers in einer Durchflußzelle (alternativ: in einer Stopped-Flow-Apparatur) inkubiert wird. Bei der Probe handelt es sich insbesondere um eine gasförmige oder eine flüssige Probe.

Es ist bevorzugt, daß keiner der Wechselwirkungspartner eine Fluoreszenzmarkierung oder ähnliches aufweist. Bei dem erfindungsgemäßen Verfahren handelt es sich vorzugsweise um ein markierungsfreies Verfahren.

Vorzugsweise handelt es sich bei den auf dem Träger immobilisierten Molekülen mindestens teilweise um Moleküle, die derart funktionalisiert sind, daß die Probenmoleküle über eine kovalente Bindung und/oder nichtkovalent über eine Affinitätsreaktion an diese anbinden bzw. sich an diese anlagern können. Hierfür geeignete Moleküle sind dem Fachmann bekannt. Die auf der Oberfläche des Trägers immobilisierten Moleküle können beispielsweise Amino-, Thiol- und Carboxylgruppen aufweisen. In besonders bevorzugten Ausführungsformen ist die Oberfläche mit den immobilisierten Molekülen derart ausgestaltet, daß sich als Probenmoleküle biologische Makromoleküle wie Proteine, Peptide, Nukleinsäuren, Lipide und Kohlenhydrate anlagern lassen.

Wenn die Probenmoleküle zu den auf dem Träger immobilisierten Molekülen kompatibel sind, sich also an diese zumindest zeitweise anlagern oder sogar eine kovalente Bindung mit diesen eingehen können, kann sich die Schichtdicke und/oder die Schichtdichte in Abhängigkeit von der Anzahl und der Art der angelagerten Moleküle ändern. Die dünne Schicht umfaßt in diesen Fällen neben den auf dem Träger immobilisierten Molekülen auch Probenmoleküle, die nun ihrerseits zumindest für eine kurze Zeit "immobilisiert" sind.

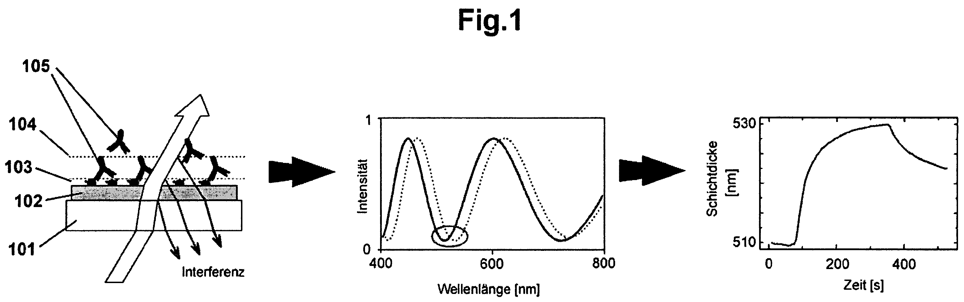

Erfindungsgemäß gilt es, die mit einem solchen Bindungs- oder Anlagerungsereignis verbundene Dickenänderung der dünnen Schicht zu bestimmen. Ändert sich die Schichtdicke, so ändert sich der Abstand zwischen der der einfallenden Strahlung zugewandten und der der einfallenden Strahlung abgewandten Begrenzungsfläche der dünnen Schicht. Aus dem damit verbundenen Gangunterschied der an diesen Begrenzungsflächen reflektierten Strahlung resultiert die eingangs bereits erwähnte Verschiebung des Interferogramms, das durch die Überlagerung der reflektierten elektromagnetischen Strahlung erzeugt wird. Die entsprechenden Zusammenhänge sind in Fig. 1 anschaulich dargestellt.

Eine dünne Schicht kann zwei oder mehr Lagen aus immobilisierten Molekülen aufweisen. In bevorzugten Ausführungsformen weist eine dünne Schicht mindestens eine erste Lage aus den oben erwähnten funktionalisierten Molekülen auf, an die Probenmoleküle über eine kovalente Bindung oder nichtkovalent anbinden können, und mindestens eine weitere Lage aus Molekülen, die zwischen der ersten Lage und dem Träger angeordnet ist. Die mindestens eine weitere Lage kann beispielsweise einer besseren Haftung der dünnen Schicht auf dem Träger oder zur Änderung der optischen Eigenschaften des Schichtsystems dienen.

Die elektromagnetische Strahlung wird bevorzugt über Einkoppelelemente auf die dünne Schicht geleitet. Zu diesen gehören bevorzugt Linsen, Linsensysteme, Spiegel oder Lichtleiter, z.B. faseroptische Wellenleiter. Ebenso können statt Einsatz von Einkoppelelementen auch entsprechende Freistrahlaufbauten realisiert werden.

Erfindungsgemäß wird mindestens ein punktförmiger oder flächenhafter Bereich der dünnen Schicht beleuchtet. Die dünne Schicht kann bei der Messung im Bezug zur einfallenden Lichtstrahlung entweder senkrecht oder in einem Winkel kleiner 90° positioniert sein.

Als mindestens eine Strahlungsquelle kann erfindungsgemäß grundsätzlich Weißlicht verwendet werden. Bevorzugt ist die elektromagnetische Strahlung jedoch an die zu erwartenden Schichtdickenänderungen angepaßt.

In besonders bevorzugten Ausführungsformen des erfindungsgemäßen Verfahrens wird die dünne Schicht mit elektromagnetischer Strahlung mit einer spektralen Bandbreite zwischen 5 nm und 50 nm, insbesondere zwischen 30 nm und 50 nm, beleuchtet. Als mindestens eine Strahlungsquelle für Licht dieser Bandbreite sind insbesondere Leuchtdioden (LED), Laserdioden und/oder Laser geeignet. Grundsätzlich kann natürlich auch eine Weißlichtquelle verwendet werden, der beispielsweise ein Monochromator nachgeschaltet ist.

Vorzugsweise erfolgt die Bestrahlung mit elektromagnetischer Strahlung aus dem Wellenlängenbereich zwischen 200 nm und 1500 nm.

Unter einem optoelektronischen Wandler soll vorliegend eine Einrichtung verstanden werden, die Licht in eine meßbare Größe, nämlich in elektrischen Strom, umwandeln kann.

Besonders bevorzugt umfaßt erfindungsgemäß der optoelektronische Wandler eine Photodiode, insbesondere eine Diode nach Art einer p-i-n-, einer p-n-, einer n-i-p-i-n- und/oder einer Schottky-Diode.

Dioden vom Typ p-i-n sind dem Fachmann bekannt. Im Unterschied zu Dioden vom Typ p-n befindet sich die p-dotierte Schicht nicht in direktem Kontakt zur n-dotierten Schicht, sondern es liegt eine schwach dotierte oder undotierte i-Schicht dazwischen.

Schottky-Dioden weichen in ihrem Aufbau ebenfalls von herkömmlichen Dioden vom Typ p-n ab. Im Gegensatz zum p-n-Übergang einer normalen Diode wird eine Schottky-Diode durch einen Halbleiter-Metall-Übergang gebildet, der ebenfalls gleichrichtende Eigenschaften besitzt, Bezüglich detaillierter Erläuterungen kann auf die einschlägigen Lehrbücher verwiesen werden.

Wie bereits erwähnt, kann die spektrale Empfindlichkeit des optoelektronischen Wandlers durch Veränderung der Auslesespannung variiert werden. Dies erfolgt vorzugsweise mit Hilfe von Steuermitteln, die den von dem optoelektronischen Wandler erzeugten Photostrom erfassen und die an den optoelektronischen Wandler angelegte Spannung gegebenenfalls erhöhen oder erniedrigen. Geeignete Steuermittel sind dem Fachmann bekannt.

Es kann grundsätzlich ein optoelektronischer Wandler verwendet werden, der Bestandteil eines externen Detektorsystems ist, das als separates Bauteil in einem Aufbau zur Durchführung von RIfS-Untersuchungen zum Einsatz kommt.

Der reflektierte Strahlungsanteil kann mit einem optoelektronischen Wandler erfaßt werden, der als Sensorschicht in und/oder auf dem oben bereits erwähnten Träger, auf dem die zu untersuchende dünne Schicht angeordnet sein kann, ausgebildet ist. Der Träger fungiert dann nicht nur als "Träger" sondern zugleich auch als Detektor. Ein separater Detektor wird entsprechende nicht mehr benötigt, so daß durch diese Maßnahme eine sehr einfache und sehr kostengünstige Durchführung von RIfS-Untersuchungen ermöglichst wird. Daneben verbessert sich durch diese Maßnahme die optische Effizienz des Verfahrens um Größenordnungen, insbesondere, da ein Detektionsstrahlengang hin zu einem separaten Detektor entfallen kann.

Besonders bevorzugt ist der optoelektronische Wandler als Dünnschichtphotodetektor ausgebildet. Die Sensorschicht weist in diesen Ausführungsformen in der Regel eine Dicke von nicht mehr als 10 µm auf. Auf den genauen Aufbau eines als Detektor fungierenden Trägers wird im folgenden noch detailliert eingegangen.

Das erfindungsgemäße Verfahren ist vielfältig einsetzbar und insbesondere auch auf spezifische Detektionsaufgaben abstimmbar. So kann die Sensitivität des Verfahrens beispielsweise gezielt variiert werden, indem das Beleuchtungsspektrum und die Spektralempfindlichkeit des optoelektronischen Wandlers synchron oder gegenläufig in Richtung größerer oder kleinerer Wellenlängen verschoben werden (das Beleuchtungsspektrum kann beispielsweise durch gegenläufige Ansteuerung des Stromes mehrerer, spektral überlappender Leuchtdioden gezielt variiert werden). Auch kann beispielsweise zuerst das Beleuchtungsspektrum zur Maximierung des Signals auf einen festen Wert verschoben werden, um dann mittels Variation der Auslesespannung einen Nullabgleich durchzuführen.

Ein als Detektor fungierender Träger für eine mittels RIfS zu untersuchende dünne Schicht ist aus dem Stand der Technik bislang noch nicht bekannt. Entsprechende ist auch ein solcher Träger bzw. Detektor Gegenstand der vorliegenden Erfindung.

Die eingangs genannte Aufgabe wird daher auch durch einen Träger gelöst, der zur Untersuchung molekularer Wechselwirkungen an und/oder in einer dünnen Schicht dient, und der zur Durchführung des erfindungsgemäßen Verfahrens gemäß einer seiner Ausgestaltungen verwendet wird.

Grundsätzlich umfaßt der Träger ein Substrat, auf dem mindestens eine Sensorschicht mit optoelektrischen Eigenschaften angeordnet ist. Der erfindungsgemäße Träger weist mehrere Schichten auf, in der genannten Reihenfolge zunächst eine Schutzschicht, insbesondere aus Siliziumdioxid, wahlweise eine hochbrechende Schicht, d.h. eine Schicht aus einem hochbrechenden Material, vorzugsweise ausgewählt aus Tantalpentoxid oder Niobpentoxid, und ein Substrat aus Glas, Kunststoff oder Metall, auf dem zumindest eine Sensorschicht mit optoelektronischen Eigenschaften angeordnet ist.

Die Dicke der Sensorschicht liegt dabei vorzugsweise im Bereich zwischen 0,1 µm und 10 µm.

Im Rahmen des erfindungsgemäßen Verfahrens fungiert die Sensorschicht als optoelektronischer Wandler.

Analog zur obigen Definition des optoelektronischen Wandlers soll vorliegend unter dem Begriff "optoelektronische Eigenschaften" die Fähigkeit verstanden werden, Licht in elektrischen Strom umwandeln zu können.

Auf seiner Oberfläche weist der erfindungsgemäße Träger eine dünne, gegebenenfalls mehrlagige Schicht aus organischen Molekülen auf, wie sie vorstehend bereits beschrieben wurde. Besonders bevorzugt weist der Träger auf seiner Oberfläche eine Lage aus immobilisierten organischen Molekülen auf, an die Proebenmoleküle unmittelbar oder nach chemischer Modifikation über eine kovalente Bindung oder nichtkovalent anbinden können.

Die zu untersuchende dünne Schicht wird vorzugsweise auf der Schutzschicht des erfindungsgemäßen Trägers aufgebracht. Gegebenenfalls bildet die Schutzschicht die Oberfläche, auf der der erfindungsgemäße Träger die dünne Schicht aus organischen Molekülen aufweist.

Die Beschaffenheit des Substrats ist relativ unkritisch, es kann beispielsweise aus Glas, Kunststoff oder auch aus Metall bestehen. Vorzugsweise ist es mindestens teilweise durchlässig für elektromagnetische Strahlung, insbesondere für Strahlung im Bereich zwischen 200 nm und 1500 nm.

Die zumindest eine Sensorschicht weist mindestens ein siliziumbasiertes Halbleitermaterial mit optoelektronischen Eigenschaften auf. Dieses ist entweder amorph, nanokristallin oder polykristallin ausgebildet und/oder es ist seiner Art nach undotiert oder dotiert und/oder nichtlegiert oder legiert.

Wenn das Halbleitermaterial der Sensorschicht nanokristallin ausgebildet ist, weist es insbesondere Kristallite mit Größen im Bereich zwischen 1 nm und 100 nm auf. Wenn es polykristallin ausgebildet ist, kann es Kristallite mit Größen von bis zu 100 µm aufweisen.

Außerdem weist die zumindest eine Sensorschicht mindestens eine durchgängige Schicht aus mindestens einem Halbleitermaterial auf und/oder mindestens eine abschnittsweise unterbrochene Schicht aus mindestens einem Halbleitermaterial. Innerhalb dieser abschnittsweise unterbrochenen Schicht liegt das Halbleitermaterial vorzugsweise in Form einzelner, insbesondere parallel zueinander angeordneter Streifen oder Rippen vor, die ein vorzugsweise regelmäßiges Muster ausbilden.

Die zumindest eine durchgängige und/oder abschnittsweise unterbrochene Schicht aus dem mindestens einen Halbleitermaterial ist nach Art einer p-i-n (oder umgekehrt, also n-i-p-), nach Art einer p-n-, nach Art einer n-i-p-i-n- und/oder nach Art einer Schottky-Diode ausgebildet.

Besonders bevorzugt weist die mindestens eine vorzugsweise durchgängige und/oder abschnittsweise unterbrochene Schicht aus dem mindestens einen Halbleitermaterial eine Abfolge von Unterschichten mit variierender optischer Bandlücke auf, insbesondere auch Abfolgen der Art p-i-n mit mehreren i-Unterschichten wie beispielsweise p-i₁-i₂-n, p-i₁-i₂-i₃-n, n-i₁-i₂-i₃-p oder n-i₁-i₂-p (oder umgekehrt).

Auch Abfolgen der Art n-i-p-i-n oder p-i-n-i-p können bevorzugt sein, wiederum sowohl mit einer als auch mit mehreren i-Unterschichten, also beispielsweise n-i₁-p-i₂-i₃-n.

Ein derartiger Aufbau gewährleistet eine gute Steuerbarkeit der Spektralempfindlichkeit der Sensorschicht.

Die optische Bandlücke der Unterschichten kann sehr einfach durch Zulegierung geeigneter Elemente variiert werden. So ist in einigen bevorzugten Ausführungsformen zumindest eine der Unterschichten mit variierender optischer Bandlücke zur Vergrößerung der Bandlücke mit Kohlenstoff legiert. In weiteren bevorzugten Ausführungsformen ist zumindest eine der Unterschichten mit variierender optischer Bandlücke zur Absenkung der Bandlücke mit Germanium legiert.

Die Unterschichten sind vorzugsweise im wesentlichen nanokristallin, amorph oder polykristallin ausgebildet. Insbesondere amorphe Unterschichten können beispielsweise durch thermische Abscheidung von Silizium aus der Gasphase hergestellt werden. In der Regel weisen sie einen geringen Anteil an Wasserstoff auf, der in die Unterschicht eingebaut ist. Gemäß einer besonders bevorzugten Ausführungsform weist die zumindest eine, vorzugsweise durchgängige und/oder abschnittsweise unterbrochene Schicht aus dem Halbleitermaterial eines erfindungsgemäßen Trägers die folgende Abfolge von Unterschichten auf:
- eine p-leitende Si:H Schicht
- eine eigenleitende Si:H Schicht
- eine eigenleitende Si:H Schicht
- eine n-leitende Si:H Schicht,
wobei Si:H jeweils einen Dünnschichthalbleiter auf der Basis von Silizium bezeichnet, der amorph oder kristallin, vorzugsweise nanokristallin, und/oder undotiert oder dotiert und/oder nichtlegiert oder mit Elementen legiert ist, die vorzugsweise ausgewählt sind aus Kohlenstoff (C), Stickstoff (N) oder Germanium (Ge).

D.h. alle diese Unterschichten sind entweder amorph ("a") oder kristallin, vorzugsweise nanokristallin ("nc"), und weisen einen Anteil an Wasserstoff ("H") auf.

Die Eigenschaften amorph oder kristallin, bzw. nanokristallin, undotiert oder dotiert, nichtlegiert oder mit Elementen legiert, die vorzugsweise, aber nicht ausschließlich ausgewählt sind aus Kohlenstoff, Stickstoff oder Germanium, können jeweils beliebig kombiniert als Eigenschaftsprofil des Dünnschichthalbleiters "Si:H" vorliegen. So kann beispielsweise amorphes SiGe, undotiert, oder ein mikrokristallines n-Typ dotiertes Silizium vorliegen, wobei diese Beispiele lediglich exemplarischen Charakter haben und die weiterhin möglichen Kombinationen nicht beschränken sollen.

Zumindest die p-leitende Schicht sowie die daran angrenzende eigenleitende Schicht weisen gemäß einer besonders bevorzugten Ausführungsform jeweils eine Kohlenstofflegierung ("C") auf.

Neben der zumindest einen vorzugsweise durchgängigen und/oder abschnittsweise unterbrochenen Schicht aus dem mindestens einen Halbleitermaterial weist die Sensorschicht in bevorzugten Ausführungsformen zumindest eine Kontaktschicht auf, die vorzugsweise unmittelbar an mindestens eine der vorzugsweise durchgängigen und/oder abschnittsweise unterbrochenen Schichten aus dem mindestens einen Halbleitermaterial angrenzt.

Die zumindest eine Kontaktschicht kann ebenfalls durchgängig und/oder abschnittsweise unterbrochen ausgebildet sein. Wenn eine Kontaktschicht als abschnittsweise unterbrochene Schicht ausgebildet ist, so weist sie bevorzugt einzelne, insbesondere parallel zueinander angeordnete Streifen oder Rippen auf, die ein vorzugsweise regelmäßiges Muster ausbilden.

Die zumindest eine Kontaktschicht kann insbesondere aus ZnO, ITO (Indium-Zinn-Oxid) und/oder aus einem Metall, insbesondere aus Aluminium, bestehen.

Vorzugsweise weist die Sensorschicht eine Kontaktschicht und eine unmittelbar mit dieser in Kontakt stehende, abschnittsweise unterbrochene Schicht aus dem mindestens einen Halbleitermaterial auf, wobei die Kontaktschicht die angrenzende Schicht an zumindest einer Seite vorzugsweise vollständig überlappt. Dadurch kann eine unerwünschte seitliche Lichteinkopplung unterbunden werden.

In bevorzugten Ausführungsformen eines erfindungsgemäßen Trägers ist auf beiden Seiten einer vorzugsweise durchgängigen und/oder abschnittsweise unterbrochenen Schicht aus dem mindestens einen Halbleitermaterial eine Kontaktschicht angeordnet. Vorzugsweise stehen die beiden Kontaktschichten dabei nicht in unmittelbarem Kontakt zueinander, so daß sie als Elektroden fungieren können, über die eine Spannung, insbesondere die oben erwähnte Auslesespannung, an die Schicht aus dem mindestens einen Halbleitermaterial angelegt werden kann.

In besonders bevorzugten Ausführungsformen ist eine abschnittsweise unterbrochene Schicht aus dem mindestens einen Halbleitermaterial zwischen einer ersten, durchgängig ausgebildeten Kontaktschicht und einer zweiten, abschnittsweise unterbrochenen Kontaktschicht angeordnet. Die zweite Kontaktschicht und die Schicht aus dem mindestens einen Halbleitermaterial sind dabei vorzugsweise im wesentlichen deckungsgleich zueinander angeordnet.

Es ist bevorzugt, daß die mindestens eine Sensorschicht des erfindungsgemäßen Trägers die folgende Schicht aufweist:
- eine erste Kontaktschicht, insbesondere aus Zinkoxid
- eine vorzugsweise durchgängige und/oder abschnittsweise unterbrochene Schicht aus dem mindestens einen Halbleitermaterial mit optoelektronischen Eigenschaften
- eine zweite Kontaktschicht, insbesondere aus Aluminium.

Die Erfindung bezieht sich auch auf ein Verfahren zur Untersuchung molekularer Wechselwirkungen an und/oder in einer dünnen Schicht mittels der reflektometrischen Interferenzspektroskopie, wie es in dem eingangs genannten Verfahren und seinen Abwandlungen bereits näher beschrieben worden ist oder die Erfindung bezieht sich auf ein solches Verfahren zur Untersuchung molekularer Wechselwirkungen an und/oder in einer dünnen Schicht mittels der reflektometrischen Interferenzspektroskopie, welches die Schritte umfaßt, Beleuchten der dünnen Schicht mit elektromagnetischer Strahlung von mindestens einer Strahlungsquelle, Erfassen eines an Begrenzungsflächen der dünnen Schicht reflektierten Strahlungsanteils mittels mindestens eines optoelektronischen Wandlers auf der Basis eines amorphen, nanokristallinen oder polykristallinen Si-basierten Halbleitermaterials, welcher die erfaßte Strahlung in einen frequenz- und intensitätsabhängigen Photostrom umwandelt, Anlegen einer Auslesespannung an den optoelektronischen Wandler zur Messung des Photostroms, wobei sich dieses modifizierte Verfahren dadurch auszeichnet, daß der reflektierte Strahlungsanteil mit einem optoelektronischen Wandler erfaßt wird, und daß dieser als Sensorschicht in dem Träger ausgebildet ist, wie er weiter oben gemäß einer seiner Ausgestaltungen beschrieben worden ist.

Gemäß einer bevorzugten Ausführungsform dieses Verfahrens wird beim Beleuchten die Wellenlänge der elektromagnetischen Strahlung variiert, insbesondere durch gegenläufiges Ansteuern von mindestens zwei unterschiedlichen, spektral überlappenden Strahlungsquellen.

Des weiteren kann die Variation der Wellenlänge der elektromagnetischen Strahlung im Hinblick auf die daraus resultierende Änderung des Photostroms synchron oder gegenläufig zur Änderung der Auslesespannung erfolgen.

Fakultativer Bestandteil des erfindungsgemäßen Trägers ist eine Antireflexschicht, die insbesondere als äußere Schicht auf der Seite des Trägers aufgebracht sein kann, die der einfallenden Strahlung zugewandt ist.

Vorzugsweise sind auch die zumindest eine, auf dem mindestens einen Halbleitermaterial basierende Schicht, die zumindest eine Kontaktschicht, die zumindest eine Schutzschicht und die Antireflexschicht mindestens teilweise durchlässig für elektromagnetische Strahlung, insbesondere für Strahlung im Bereich zwischen 200 nm und 1500 nm.

Weitere Merkmale der Erfindung ergeben sich aus den Fig. der Zeichnung in Verbindung mit den Unteransprüchen. Hierbei können einzelne Merkmale jeweils für sich ober zu mehreren in Kombination miteinander bei einer Ausführungsform der Erfindung verwirklicht sein. Die beschriebenen bevorzugten Ausführungsformen dienen lediglich zur Erläuterung und zum besseren Verständnis der Erfindung und sind in keiner Weise einschränkend zu verstehen.

### Figurenbeschreibung

- Fig. 1: veranschaulicht das der Erfindung zugrundeliegende Prinzip, dem zufolge ein Bindungsereignis über die optische Untersuchung einer dünnen Schicht verfolgt werden kann.
- Fig. 2: zeigt eine Anordnung für ein erfindungsgemäßes Verfahren zur Untersuchung einer dünnen Schicht mittels einer RIfS-Messung, wobei ein erfindungsgemäßer Träger als separater Detektor zum Einsatz kommt.
- Fig. 3: zeigt eine Ausführungsform eines erfindungsgemäßen Trägers zur Untersuchung molekularer Wechselwirkungen an und/oder in einer dünnen Schicht.
- Fig. 4: zeigt einen Vergleich eines aus dem Stand der Technik bekannten Trägers für eine dünne Schicht und eines als Detektor fungierenden erfindungsgemäßen Trägers für eine dünne Schicht.
- Fig. 5: zeigt eine Ausführungsform einer Sensorschicht eines erfindungsgemäßen Trägers (Querschnitt).
- Fig. 6: zeigt schematisch eine Anordnung zur Ansteuerung eines erfindungsgemäßen Trägers.

**Fig. 1** veranschaulicht das der Erfindung zugrundeliegende und aus dem Stand der Technik bereits bekannte Prinzip, dem zufolge ein Bindungsereignis über die optische Untersuchung einer dünnen Schicht verfolgt werden kann. Dargestellt ist der Träger **101,** auf dem eine zu untersuchende dünne Schicht umfassend eine Lage **103** aus funktionalen immobilisierten Molekülen und eine weitere Lage **102** aus Molekülen, die zwischen der Lage **103** und dem Träger **101** angeordnet ist (schematisch dargestellt). Der Träger **101** kann z.B. aus Glas, Kunststoff oder aus Metall bestehen. Die Lage **102** dient insbesondere der besseren Haftung der dünnen Schicht auf dem Träger. An die funktionalen, immobilisierten Moleküle der Lage **103** können Probenmoleküle **105** anbinden. In der Folge wächst die Dicke der dünnen Schicht an, der Abstand ihrer oberen Begrenzungsfläche zur Phasengrenze zwischen dünner Schicht und Träger (bzw. zur unteren Begrenzungsfläche der dünnen Schicht) wird größer. Von unten eingestrahltes Licht wird nach Anbindung der Probenmoleküle **105** nunmehr auch an der Begrenzungsfläche **104** reflektiert. Da es bis zur Begrenzungsfläche **104** eine größere Weglänge zurücklegen muß, resultiert eine Verschiebung des Interferogramms, das durch die Überlagerung der reflektierten elektromagnetischen Strahlung erzeugt wird (die gestrichelte Linie entspricht dem (Ausgangs-) Interferenzspektrum, die durchgezogene Linie der nach Anlagerung der Proberunoleküle **105** gemessenen sinusförmigen Detektionskurve). Diese Verschiebung kann zeitaufgelöst bestimmt werden, was genaue Rückschlüsse auf die Schichtdickenänderung und damit auf Wechselwirkungen zwischen den Probenmolekülen **105** und den funktionalen, immobilisierten Molekülen in der Lage **103** zuläßt. Der Verlauf einer Schichtdickenänderung ist im Diagramm rechts beispielhaft aufgetragen. Bis ca. 350 s wurde eine Schichtdickenzunahme beobachtet, danach wieder eine Abnahme. Dies entspricht dem typischen Verlauf einer Bindungsreaktion von zu detektierenden Antikörpern an auf dem Träger immobilisierten Antigenen und deren anschließende Ablösung.

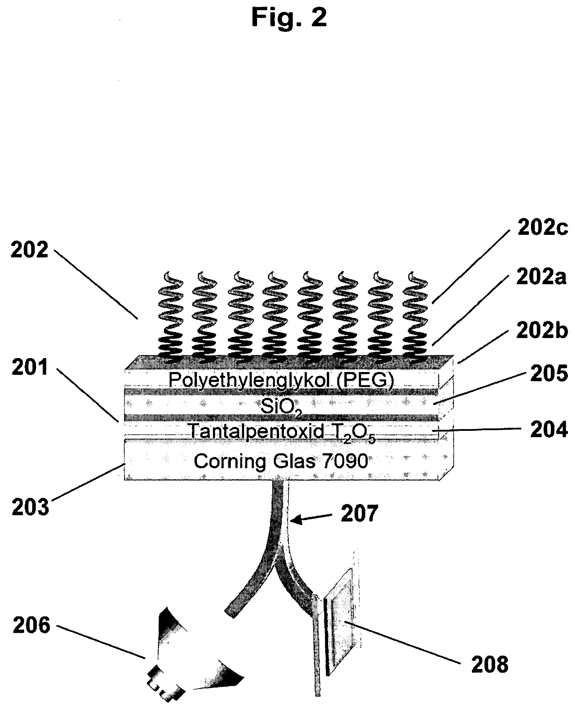

**Fig. 2** zeigt eine Anordnung für ein erfindungsgemäßes Verfahren zur Untersuchung einer dünnen Schicht mittels einer RIfS-Messung. Die Anordnung umfaßt einen (aus dem Stand der Technik bekannten) Träger **201,** auf dem die zu untersuchende dünne Schicht **202** angeordnet ist. Diese umfaßt eine Lage aus funktionalen Molekülen **202a,** die über eine weitere Lage aus Polyethylenglykolmolekülen **202b** auf der Oberfläche des Trägers immobilisiert sind. Darüber hinaus umfaßt sie eine Lage aus Probenmolekülen **202c,** die an die funktionalen Molekülen **202a** angebunden oder angelagert sind. Der Träger **201** umfaßt ein Substrat aus Corning-Glas **203,** eine Schicht aus hochbrechendem Material (Tantalpentoxid) **204** und eine Schutzschicht aus Siliziumdioxid **205,** die die Oberfläche des Trägers **201** ausbildet, auf der die zu untersuchende dünne Schicht **202** aufgebracht ist. Eine Strahlungsquelle **206** (links unten) liefert die zur Untersuchung benötigte elektromagnetische Strahlung, die über ein Einkoppelelement (einen Y-Lichtleiter) **207** auf die dünne Schicht **202** geleitet wird. Ein an den Begrenzungsflächen der dünnen Schicht reflektierter Strahlungsanteil wird wiederum über den Y-Lichtleiter **207** einem separaten Detektor **208** zugeführt. Als Detektor **208** kann vorliegend ein erfindungsgemäßer Träger zum Einsatz kommen. Dieser umfaßt eine Sensorschicht auf der Basis eines amorphen, Si-basierten Halbleitermaterials, das optoelektronische Eigenschaften hat und die reflektierte Strahlung in einen Photostrom umwandeln kann. Die spektrale Empfindlichkeit des optoelektronischen Wandlers kann durch Veränderung der Auslesespannung variiert werden, so daß ein im wesentlichen konstanter Photostrom erhalten wird. Aus dem Verlauf der Auslesespannung können anschließend Parameter abgeleitet werden, welche die zu untersuchenden Wechselwirkungen an und/oder in der dünnen Schicht **202** kennzeichnen. Alternativ können aus der Änderung des Photostroms auch unmittelbar Parameter abgeleitet werden, die Rückschlüsse auf erfolgte Änderungen der Dicke der dünnen Schicht **202** und somit auf Wechselwirkungen an und/oder in der dünnen Schicht **202** zulassen.

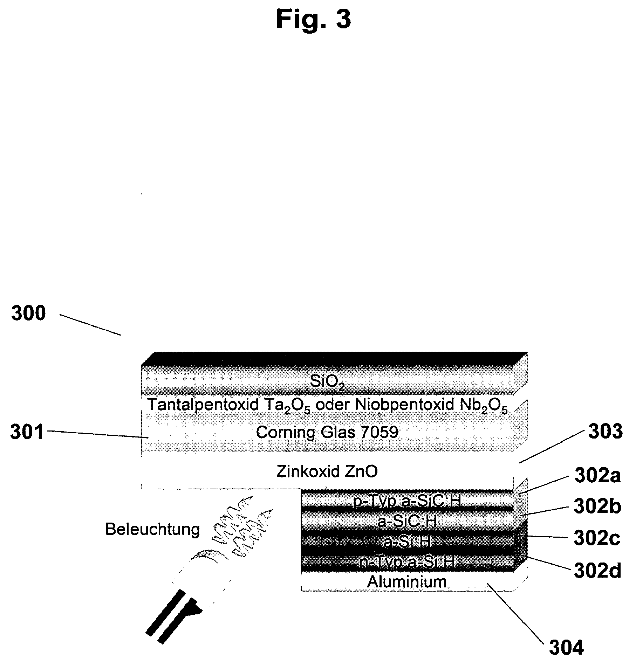

**Fig. 3** zeigt eine Ausführungsform eines erfindungsgemäßen Trägers **300** zur Untersuchung molekularer Wechselwirkungen an und/oder in einer dünnen Schicht. Die Schichten oberhalb des Substrates **301** aus Corning Glas entsprechen der Anordnung in Fig. 2, während die Schichten unterhalb des Glassubstrates **301** einer Sensorschicht entsprechen, wie sie vorab bereits beschrieben wurde. Die Sensorschicht umfaßt eine Schicht mit optoelektronischen Eigenschaften **302,** basierend auf einem Halbleitermaterial, eine erste Kontaktschicht aus Zinkoxid **303** und eine zweite Kontaktschicht aus Aluminium **304.** Die Schicht mit optoelektronischen Eigenschaften **302** weist eine Abfolge mit einer p-leitenden a-SiC:H Schicht **302a,** einer eigenleitenden a-SiC:H Schicht **302b,** einer eigenleitenden aSi:H Schicht **302c** und einer n-leitenden a-Si:H Schicht **302d** auf. Die Gesamtdicke der Sensorschicht beträgt ca. 0,5 - 3 µm.

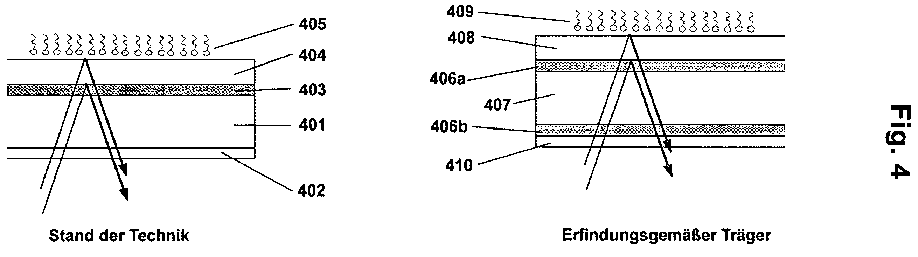

**Fig. 4** zeigt einen Vergleich eines aus dem Stand der Technik bekannten Trägers für eine dünne Schicht mit einem als Detektor fungierenden erfindungsgemäßen Träger für eine dünne Schicht. Der aus dem Stand der Technik bekannte Träger entspricht im wesentlichen dem in Fig. 1 abgebildeten Träger. Er weist ein Substrat aus Glas **401,** eine Antireflexschicht **402,** eine Schicht aus hochbrechendem Material **403** und eine Schutzschicht aus Siliziumdioxid **404** auf, auf der die zu untersuchende dünne Schicht **405** angeordnet ist. Rechts ist ein erfindungsgemäßer Träger dargestellt, der eine Sensorschicht **406** (ggf. auch zwei Sensorschichten) mit optoelektronischen Eigenschaften aufweist. Wie der aus dem Stand der Technik bekannte Träger umfaßt er ebenfalls ein Substrat aus Glas **407** und eine Schutzschicht aus Siliziumdioxid **408,** auf der die zu untersuchende dünne Schicht **409** angeordnet ist. Die Sensorschicht ist entweder oberhalb **(406a),** unterhalb **(406b)** oder auf beiden Seiten des Substrates **407** angeordnet. Die unterhalb von Sensorschicht **406b** angeordnete Antireflexschicht **410** ist optional. Aufgrund ihres hohen Brechungsindex kann die Sensorschicht **406a** auch gleichzeitig die Funktion der interferenzverstärkenden, hochbrechenden Schicht **403** in der aus dem Stand der Technik bekannten Anordnung übernehmen und diese ersetzen.

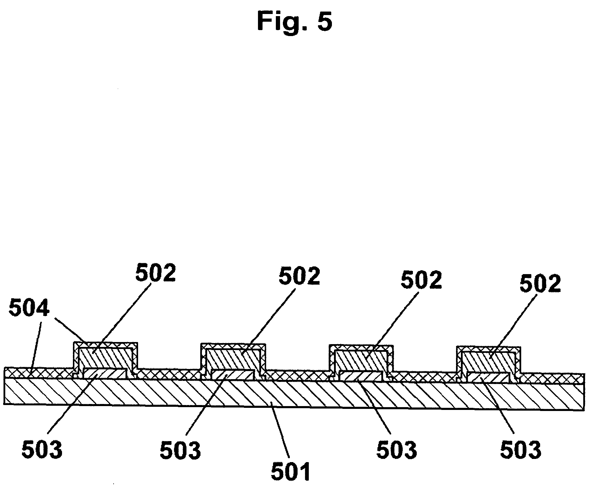

**Fig. 5** zeigt schematisch eine Ausführungsform einer Sensorschicht eines erfindungsgemäßen Trägers (Querschnitt). Die Sensorschicht befindet sich auf einem Glassubstrat **501.** Auf diesem ist ein Halbleitermaterial mit optoelektronischen Eigenschaften in Form von länglichen, parallel zueinander ausgerichteten, senkrecht zur Bildebene verlaufenden Streifen **502** angeordnet. Die Streifen sind in einer Ebene angeordnet und bilden in dieser eine abschnittsweise unterbrochene Schicht aus. Zwischen den Streifen **502** und dem Substrat **501** sind - ebenfalls in Form von senkrecht zur Bildebene verlaufenden Streifen - die Kontakte **503** ausgebildet. Auch die Kontaktstreifen sind in einer Ebene angeordnet und bilden in dieser eine abschnittsweise unterbrochene Schicht aus. Die Kontakte **503** werden von den Streifen **502** im wesentlichen vollständig überdeckt. Sie sind über einen Sammelkontakt (nicht dargestellt) verbunden. Die abschnittsweise unterbrochene Schicht aus dem Halbleitermaterial ist von einer durchgängigen, vorzugsweise undurchsichtigen Kontaktschicht **504** überzogen. Die Schicht **504** deckt die Streifen **502** dabei insbesondere auch seitlich ab. Die Kontaktschicht **504** und die Kontaktstreifen **503** stehen nicht in unmittelbarem Kontakt miteinander. Über die Kontaktschicht **504** und die Kontaktstreifen 503 (bzw. einen nicht dargestellten Sammelkontakt) kann zur Durchführung des erfindungsgemäßen Verfahrens eine Spannung, insbesondere eine Auslesespannung, an die Streifen **502** aus dem Halbleitermaterial mit optoelektronischen Eigenschaften angelegt werden.

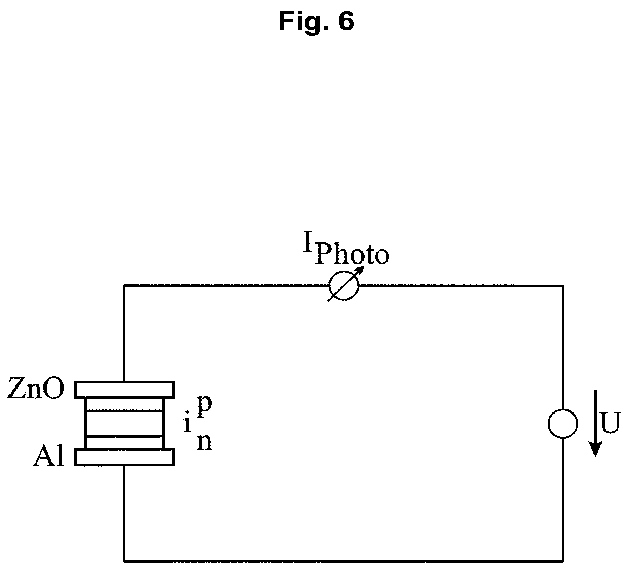

**Fig. 6** zeigt schematisch eine Anordnung zur Ansteuerung eines erfindungsgemäßen Trägers, insbesondere im Rahmen des erfindungsgemäßen Verfahrens. Eine Schicht aus dem mindestens einen Halbleitermaterial, die nach Art einer n-i-p-Diode ausgebildet ist, ist zwischen einer Kontaktschicht aus Zinkoxid und einer Kontaktschicht aus Aluminium angeordnet. Die Kontaktschichten können als Elektroden fungieren, so daß an das dazwischenliegende Halbleitermaterial eine Auslesespannung angelegt werden kann. Durch Variation der Auslesespannung kann die spektrale Empfindlichkeit des optoelektronischen Wandlers variiert werden, so daß ein im wesentlicher konstanter Photostrom erhalten wird.

## Patentansprüche

1. Verfahren zur Untersuchung molekularer Wechselwirkungen an und/oder in einer auf einem Träger angeordneten dünnen Schicht mittels der Reflektometrischen Interferenzspektroskopie (RIfS), umfassend die Schritte
(1) Beleuchten der dünnen Schicht mit elektromagnetischer Strahlung von mindestens einer Strahlungsquelle,
(2) Erfassen eines an Begrenzungsflächen der dünnen Schicht reflektierten Strahlungsanteils mittels mindestens eines optoelektronischen Wandlers auf der Basis eines amorphen, nanokristallinen oder polykristallinen Si-basierten Halbleitermaterials, welcher die erfaßte Strahlung in einen frequenz- und intensitätsabhängigen Photostrom umwandelt,
(3) Anlegen einer Auslesespannung an den optoelektronischen Wandler zur Messung des Photostroms,
**dadurch gekennzeichnet, daß** die spektrale Empfindlichkeit des optoelektronischen Wandlers durch Veränderung der Auslesespannung variiert wird, so daß ein im wesentlichen konstanter Photostrom erhalten wird, wobei aus der Auslesespannung Parameter abgeleitet werden, welche die zu untersuchenden Wechselwirkungen an und/oder in der dünnen Schicht kennzeichnen.

2. Verfahren nach Anspruch 1, **dadurch gekennzeichnet, daß** die auf dem Träger angeordnete dünne Schicht der einfallenden Strahlung abgewandt ist.

3. Verfahren nach einem der Ansprüche 1 bis 2, **dadurch gekennzeichnet, daß** die zu untersuchenden molekularen Wechselwirkungen physikalische, chemische und/oder biochemische Reaktionen, Erkennungs-, Bindungs- und/oder Anlagerungsereignisse sind.

4. Verfahren nach einem der Ansprüche 1 bis 3, **dadurch gekennzeichnet, daß** die dünne Schicht mit elektromagnetischer Strahlung mit einer spektralen Bandbreite zwischen 5 nm und 50 nm, insbesondere zwischen 30 nm und 50 nm, beleuchtet wird.

5. Verfahren nach einem der Ansprüche 1 bis 4, **dadurch gekennzeichnet, daß** der optoelektronische Wandler eine Photodiode, insbesondere eine Photodiode nach Art einer p-i-n-, einer p-n-, einer n-i-p-i-n- und/oder einer Schottky-Diode, umfaßt.

6. Träger für eine dünne Schicht zur Untersuchung molekularer Wechselwirkungen an und/oder in der dünnen Schicht, zur Durchführung des Verfahrens nach einem der Ansprüche 1 bis 5, wobei der Träger mehrere Schichten aufweist, in der genannten Reihenfolge zunächst eine Schutzschicht und ein Substrat aus Glas, Kunststoff oder Metall, auf dem zumindest eine Sensorschicht mit optoelektronischen Eigenschaften angeordnet ist, welche zumindest ein amorphes, nanokristallines oder polykristallines und/oder undotiertes oder dotiertes und/oder nichtlegiertes oder legiertes, siliziumbasiertes Halbleitermaterial mit optoelektronischen Eigenschaften aufweist und zumindest eine durchgängige Schicht und/oder zumindest eine abschnittsweise unterbrochene Schicht aus dem zumindest einen Halbleitermaterial umfaßt, die nach Art einer p-i-n-, nach Art einer n-i-p-, nach Art einer p-i-n-i-p-, nach Art einer n-i-p-i-n- und/oder nach Art einer Schottky-Diode ausgebildet ist, mit einer Abfolge von Unterschichten mit variierender optischer Bandlücke, die eine Schichtfolge mit einer oder mehreren i-Unterschichten aufweist.

7. Träger nach Anspruch 6, **gekennzeichnet durch** die Abfolge von Unterschichten in Form von:
- einer p-leitenden Si:H-Schicht,
- einer eigenleitenden Si:H-Schicht,
- einer eigenleitenden Si:H-Schicht und
- einer n-leitenden Si:H-Schicht.

8. Träger nach Anspruch 6 oder 7, **dadurch gekennzeichnet, daß** zumindest eine der Unterschichten mit Kohlenstoff und/oder Germanium dotiert ist und daß die Elemente für die Legierung ausgewählt sind aus Kohlenstoff, Stickstoff oder Germanium.

9. Träger nach einem der Ansprüche 6 bis 8, **dadurch gekennzeichnet, daß** die zumindest eine Sensorschicht mindestens eine Kontaktschicht aufweist, die unmittelbar an die mindestens eine durchgängige und/oder abschnittsweise unterbrochene Schicht aus dem mindestens einen Halbleitermaterial angrenzt.

10. Träger nach Anspruch 9, **dadurch gekennzeichnet, daß** die zumindest eine Kontaktschicht aus ZnO, ITO (Indium-Zinn-Oxid) und/oder einem Metall besteht.

11. Träger nach Anspruch 9 oder 10, **dadurch gekennzeichnet, daß** die zumindest eine Sensorschicht eine Kontaktschicht und eine unmittelbar an diese angrenzende, abschnittsweise unterbrochene Schicht aus dem mindestens einen Halbleitermaterial aufweist, wobei die Kontaktschicht die angrenzende, abschnittsweise unterbrochene Schicht an mindestens einer Seite überlappt.

12. Träger nach einem der Ansprüche 9 bis 11, **dadurch gekennzeichnet, daß** die mindestens eine Sensorschicht die folgende Schichtabfolge aufweist:
- eine Kontaktschicht, bevorzugt aus Zinkoxid
- eine vorzugsweise durchgängige und/oder abschnittsweise unterbrochene Schicht aus dem mindestens einen Halbleitermaterial mit optoelektronischen Eigenschaften
- eine metallische Kontaktschicht, bevorzugt aus Aluminium.

13. Verfahren zur Untersuchung molekularer Wechselwirkungen an und/oder in einer dünnen Schicht nach einem der Ansprüche 1 bis 6 oder nach dem Oberbegriff von Anspruch 1, **dadurch gekennzeichnet, daß** der reflektierte Strahlungsanteil mit einem optoelektronischen Wandler erfaßt wird, der als Sensorschicht in einem Träger nach einem der Ansprüche 6 bis 12 ausgebildet ist.

14. Verfahren nach Anspruch 13, **dadurch gekennzeichnet, daß** beim Beleuchten die Wellenlänge der elektromagnetischen Strahlung variiert wird, insbesondere durch gegenläufiges Ansteuern von mindestens zwei unterschiedlichen, spektral überlappenden Strahlungsquellen.

15. Verfahren nach Anspruch 14, **dadurch gekennzeichnet, daß** die Variation der Wellenlänge der elektromagnetischen Strahlung im Hinblick auf die daraus resultierende Änderung des Photostroms synchron oder gegenläufig zur Änderung der Auslesespannung erfolgt.

## Claims

1. Method to investigate molecular interactions on and/or in a thin layer arranged on a carrier, by means of reflectometric interference spectroscopy (RifS), consisting of the stages
(1) illuminating the thin layer with electromagnetic radiation from at least one radiation source,
(2) detecting a part of the beam reflected from the boundary-surfaces of the thin layer by at least one optoelectronic transducer based on an amorphous, nanocrystalline or polycrystalline Si-based semiconductor material which converts the detected radiation into a frequency- and intensity-dependent photocurrent,
(3) applying a readout voltage to the optoelectronic transducer to measure the photocurrent,
**characterised in that** the spectral sensitivity of the optoelectronic transducer is varied by changing the readout voltage, so that an essentially constant photocurrent is obtained, and where parameters are derived from the read-out voltage which identify the interactions that are to be examined on and/or in the thin layer.

2. Method according to Claim 1, **characterised in that** the thin layer arranged on the carrier is facing away from the incident beam.

3. Method according to one of the Claims 1 to 2, **characterised in that** the molecular interactions to be examined are physical, chemical and/or biochemical reactions, identification events, binding events and/or adsorption events.

4. Method according to one of the Claims 1 to 3, **characterised in that** the thin layer is illuminated by electromagnetic radiation with a spectral bandwidth from 5 nm to 50 nm, in particular from 30 nm to 50 nm.

5. Method according to one of the Claims 1 to 4, **characterised in that** the optoelectronic transducer includes a photodiode, in particular a photodiode of the type p-i-n-, or p-n-, or n-i-p-i-n- and/or a Schottky diode.

6. Carrier for a thin layer to investigate molecular interactions on and/or in the thin layer, to perform the method according to one of the Claims 1 to 5, where the carrier has several layers, in the said order: first a protective layer and a substrate made of glass, plastic or metal, on which at least one sensor layer with optoelectronic properties is arranged, which has at least one amorphous nanocrystalline or polycrystalline and/or undoped or doped and/or non-alloyed or alloyed, silicon-based semiconductor material with optoelectronic properties, and at least one continuous layer and/or at least one sectionally discontinuous layer, made of at least one semiconductor material, that is formed as a photodiode of the type p-i-n-, or n-i-p-, or p-i-n-i-p-, or n-i-p-i-n-and/or a Schottky diode, with a sequence of sublayers of varying optical bandgaps, which has a layer sequence with one or more i-sublayers.

7. Carrier according to Claim 6, **characterised in that** the sequence of sublayers is in the form of:
- a p-conducting Si:H layer,
- an intrinsic Si:H layer,
- an intrinsic Si:H layer, and
- an n-conducting Si:H layer.

8. Carrier according to Claim 6 or 7, **characterised in that** at least one of the sublayers is doped with carbon and/or germanium, and that the elements for the alloy are selected from carbon, nitrogen or germanium.

9. Carrier according to one of the Claims 6 to 8, **characterised in that** the at least one sensor layer has at least one contact layer, which is directly adjacent to the at least one continuous and/or sectionally discontinuous layer made of the at least one semiconductor material.

10. Carrier according to Claim 9, **characterised in that** the at least one contact layer is made of ZnO, ITO (indium tin oxide) and/or a metal.

11. Carrier according to Claim 9 or 10, **characterised in that** the at least one sensor layer has a contact layer and a sectionally interrupted layer directly adjacent to this made of at least one semiconductor material, where the contact layer overlaps the adjacent sectionally discontinuous layer on at least one side.

12. Carrier according to one of the Claims 9 to 11, **characterised in that** the at least one sensor layer has the following sequence of layers:
- a contact layer, preferably made of zinc oxide,
- a preferably continuous and/or sectionally discontinuous layer made of the at least one semiconductor material with optoelectronic properties,
- a metallic contact layer, preferably made of aluminium.

13. Method to investigate molecular interactions on and/or in a thin layer according to one of the Claims 1 to 6 or according to the main concept of Claim 1, **characterised in that** the reflected part of the beam is recorded by an optoelectronic transducer which is designed as a sensor layer in a carrier according to one of the Claims 6 to 12.

14. Method according to Claim 13, **characterised in that** when applying illumination the wavelength of the electromagnetic radiation is varied, in particular by opposite driving of at least two different, spectrally overlapping sources of radiation.

15. Method according to Claim 14, **characterised in that** the variation of the wavelength of the electromagnetic radiation is performed synchronously or opposite to the change in the readout voltage, to observe the resulting change of the photocurrent.

## Revendications

1. Procédé pour analyser des interactions moléculaires au niveau d'une couche mince et/ou dans une couche mince disposée sur un support au moyen d'une spectroscopie interférentielle par réflectométrie (RIfS), comprenant les étapes suivantes :
(1) l'éclairage de la couche mince avec un rayonnement électromagnétique d'au moins une source de rayonnement,
(2) la détection d'une partie de rayonnement réfléchie au niveau de surfaces de délimitation de la couche mince au moyen d'au moins un convertisseur optoélectronique sur la base d'un matériau semi-conducteur amorphe, nanocristallin ou poly-cristallin à base de Si, lequel convertit le rayonnement détecté en un courant photoélectrique dépendant de la fréquence et de l'intensité,
(3) l'application d'une tension de lecture au convertisseur optoélectronique pour la mesure du courant photoélectrique,
**caractérisé en ce que** l'on fait varier la sensibilité spectrale du convertisseur optoélectronique en modifiant la tension de lecture de manière à obtenir un courant photoélectrique sensiblement constant, dans lequel des paramètres sont déduits de la tension de lecture, lesquels caractérisent les interactions à analyser au niveau de la couche mince et/ou à l'intérieur de celle-ci.

2. Procédé selon la revendication 1, **caractérisé en ce que** la couche mince disposée sur le support tourne le dos au rayonnement incident.

3. Procédé selon l'une des revendications 1 à 2, **caractérisé en ce que** les interactions moléculaires à analyser sont des réactions physiques, chimiques et/ou biochimiques, des évènements de reconnaissance, de liaison et/ou de fixation par addition.

4. Procédé selon l'une des revendications 1 à 3, **caractérisé en ce que** la couche mince est éclairée avec un rayonnement électromagnétique avec une largeur de bande spectrale comprise entre 5 nm et 50 nm, en particulier entre 30 nm et 50 nm.

5. Procédé selon l'une des revendications 1 à 4, **caractérisé en ce que** le convertisseur optoélectronique comprend une photodiode, en particulier une photodiode du genre d'une diode pi-n, p-n, n-i-p-i-n et/ou d'une diode de Schottky.

6. Support pour une couche mince pour l'analyse d'interactions moléculaires au niveau de la couche mince et/ou à l'intérieur de la couche mince pour la mise en oeuvre du procédé selon l'une des revendications 1 à 5, dans lequel le support présente plusieurs couches dans l'ordre suivant mentionné : tout d'abord une couche de protection et un substrat en verre, plastique ou métal, sur lequel est disposé au moins une couche de capteur avec des propriétés optoélectroniques, laquelle présente au moins un matériau semi-conducteur amorphe, nanocristallin ou poly-cristallin et/ou non dopé ou dopé et/ou non allié ou allié à base de silicium avec des propriétés optoélectroniques et au moins une couche continue et/ou au moins une couche partiellement interrompue en l'au moins un matériau semi-conducteur, laquelle est réalisée à la manière d'une diode p-i-n, à la manière d'une diode p-i-n-i-p, à la manière d'une diode n-i-p-i-n et/ou à la manière d'une diode de Schottky, avec une succession de sous-couches avec des ruptures de bande variant optiquement, lesquelles présentent une succession de couches avec une ou plusieurs sous-couches i.

7. Support selon la revendication 6, **caractérisé par** la succession de sous-couches sous la forme suivante :
- une couche de Si:H à conduction p,
- une couche de Si:H à conduction intrinsèque,
- une couche de Si:H à conduction intrinsèque, et
- une couche de Si:H à conduction n.

8. Support selon la revendication 6 ou 7, **caractérisé en ce qu'**au moins l'une des sous-couches est dopée avec du carbone et/ou du germanium et **en ce que** les éléments pour l'alliage sont sélectionnés parmi du carbone, de l'azote ou du germanium.

9. Support selon l'une des revendications 6 à 8, **caractérisé en ce que** l'au moins une couche de capteur présente au moins une couche de contact, laquelle est directement adjacente à l'au moins une couche continue et/ou partiellement interrompue en l'au moins un matériau semi-conducteur.

10. Support selon la revendication 9, **caractérisé en ce que** l'au moins une couche de contact se compose de ZnO, d'ITO (oxyde d'indium et d'étain) et/ou d'un métal.

11. Support selon la revendication 9 ou 10, **caractérisé en ce que** l'au moins une couche de capteur présente une couche de contact et une couche partiellement interrompue directement adjacente à celle-ci en l'au moins un matériau semi-conducteur, dans lequel la couche de contact chevauche la couche adjacente partiellement interrompue, au moins sur un côté.

12. Support selon l'une des revendications 9 à 11, **caractérisé en ce que** l'au moins une couche de capteur présente la succession de couches suivante :
- une couche de contact, de préférence en oxyde de zinc,
- une couche de préférence continue et/ou partiellement interrompue en l'au moins un matériau semi-conducteur avec des propriétés optoélectroniques,
- une couche de contact métallique, de préférence en aluminium.

13. Procédé pour l'analyse d'interactions moléculaires au niveau d'une couche mince et/ou à l'intérieur d'une couche mince selon l'une des revendications 1 à 6 ou selon le préambule de la revendication 1, **caractérisé en ce que** la partie de rayonnement réfléchie est détectée avec un convertisseur optoélectronique, lequel est réalisé en tant que couche de capteur dans un support selon l'une des revendications 6 à 12.

14. Procédé selon la revendication 13, **caractérisé en ce que**, lors de l'éclairage, on fait varier la longueur d'onde du rayonnement électromagnétique, en particulier grâce à une commande en opposition d'au moins deux sources de rayonnement différentes à superposition spectrale.

15. Procédé selon la revendication 14, **caractérisé en ce que** la variation de la longueur d'onde du rayonnement électromagnétique, compte tenu de la modification du courant photoélectrique qui en résulte, s'effectue de manière synchrone ou en opposition par rapport à la modification de la tension de lecture.
